# NEUE EUROPÄISCHE PATENTSCHRIFT

(11) **EP 0 867 713 B2**
(45) Veröffentlichungstag und Bekanntmachung des Hinweises auf die Entscheidung über den Einspruch: **22.10.2014**
(45) Hinweis auf die Patenterteilung: 28.12.2005
(21) Anmeldenummer: 98101115.8
(22) Anmeldetag: 23.01.1998
(51) Int. Cl.: G01N 27/12, G01N 33/00

(54) **Sonde zum Messen von flüchtigen Bestandteilen in einer wässrigen Lösung**
Probe for measuring volatile components in an aqueous solution
Sonde pour la mesure des composants volatiles dans une solution aqueuse

(30) Priorität: 03.02.1997 DE 19703744; 03.02.1997 DE 29701652 U
(43) Veröffentlichungstag der Anmeldung: 30.09.1998
(73) Patentinhaber: Sellmer-Wilsberg, Sylvia, Dr., 53619 Rheinbreitbach (DE); Wilsberg, Hans-Werner, 53619 Rheinbreitbach (DE)
(72) Erfinder: Sellmer-Wilsberg, Sylvia, Dr., 53619 Rheinbreitbach (DE); Wilsberg, Hans-Werner, 53619 Rheinbreitbach (DE)
(74) Vertreter: Müller-Gerbes, Margot

(56) Entgegenhaltungen:
- EP-A- 0 054 537
- DE-A- 3 126 648
- DE-A- 19 604 606
- DE-C- 3 126 648
- US-A- 4 821 585
- 'Biotechnology letters', Bd. 5, teil 8 1983 Seiten 509 - 514
- 'Unitronic Prospekt: Figaro Gas Sensors', April 1985

## Beschreibung

Die Erfindung befaßt sich mit der Verwendung einer Sonde zum Messen von flüchtigen Bestandteilen in einer wässrigen Lösung, wie der Bestimmung der Alkoholkonzentration einer wässrigen Lösung, mit einem Sondenkörper mit einem durchgehenden Lumen und einer quer zum Lumen verlaufend angeordneten Membran, die das Lumen nach außen hin abtrennt und mit einem innerhalb des Lumens mit Abstand zu der Membran in einem Gehäuse angeordneten Halbleitergassensor, wobei eine von der äußeren Atmosphäre und Umgebung abgetrennte mit Luft gefüllte Meßkammer innerhalb des Gehäuses bzw. bis zur Membran hin ausgebildet ist und der Sensor auf die durch die Membran in die Meßkammer permeierenden Gase mit einer Änderung des elektrischen Widerstandes anspricht.

Eine Sonde zur Messung organischer Lösemittel in einer Flüssigkeit unter Verwendung eines Halbleitergassensors in einer geschlossenen Meßkammer mit einer flüssigkeitsundurchlässigen Membran, durch welche die Gase aus der zu messenden Flüssigkeit in die Meßkammer diffundieren, ist aus der DE 31 26 648 C2 bekannt. Die Meßkammer ist als geschlossenes System aufgebaut, wobei bei abnehmender Konzentration der Meßkomponente in der zu messenden Flüssigkeit das in der Meßkammer angereicherte Gas wieder durch die Membran in die Flüssigkeit zurückdiffundiert. Bei dieser Sonde erfolgt ein Gasaustausch durch Diffusion ausschließlich über die Membran in beiden Richtungen. Diese Sonde arbeitet sehr langsam und in der Weise, daß bei Auftreten eines Konzentrationsunterschiedes zwischen Meßkammer und Flüssigkeit ein Stofftransport z. B. in Richtung Meßkammer eintritt. Durch die sich dadurch ergebende zunehmende Angleichung der Partialdrücke infolge des in die Meßkammer eindiffundierenden Gases vermindert sich die Diffusionsgeschwindigkeit, und die Konzentration in der Meßkammer und damit das Meßsignal nähern sich langsam asymptotisch dem Gleichgewichtszustand. Erst nach Rückdiffusion der Gase aus der Meßkammer in die Flüssigkeit kann ein erneutes Eindiffundieren erfolgen. Die Sonde ist für den Einsatz in technischen Prozessen infolge des nur sehr langsamen Gasumlaufs durch Rückdiffusion durch die Membran nicht geeignet.

Andere Sonden zur Entnahme von flüchtigen Komponenten aus Flüssigkeiten oder Gasen, beispielsweise zwecks Konzentrationsbestimmung, die mit einer Permeationsmembran sowie einem Sensor arbeiten, sind beispielsweise aus der EP-A 0174 417 und der EP-A 0054 537 bekannt geworden. Bei diesen bekannten Sonden werden Permeationsmembrane auf Basis eines schlauchförmigen Silikonkörpers eingesetzt, durch welche die zu messenden flüchtigen Komponenten in den Flüssigkeiten entsprechend ihrer Konzentration mit unterschiedlichen Geschwindigkeiten permeieren und dann auf den Sensor treffen, welcher auf Grund seiner elektrischen Eigenschaften in Abhängigkeit von der Konzentration der zu messenden Komponenten seinen Widerstand ändert und entsprechende Meßsignale abgibt. Zur Erhöhung der Meßleistung wird die schlauchförmige Permeationsmembran mit einem Trägergas gespült.

Bei diesen bekannten Sonden erweist es sich als nachteilig, daß durch Anwendung der schlauchförmigen Silikonmembranen, die keine stoffspezifische Trennwirkungen erzeugen, nicht nur der gewünschte zu messende Stoff/Gas aus der Flüssigkeit abgetrennt und gemessen wird, sondern auch weitere Gase und Stoffe das Meßergebnis in bezug auf diesen einen gewünschten Stoff verfälschen.

Darüber hinaus zeigen die bekannten Silikonmembranen recht begrenzte Diffusionsgeschwindigkeiten insbesondere auch in bezug auf Alkohol, so daß sich die Notwendigkeit ergibt, eine sehr große Membranfläche zur Verfügung zu stellen.

Es wurde daher gemäß DE 196 04 606 A1 bereits eine Meßvorrichtung für einen von wenigstens zwei flüchtigen Bestandteilen einer Flüssigkeit vorgeschlagen, bei der eine aus wenigstens zwei Schichten mit unterschiedlichen Durchdringungswiderstand aufgebaute Permeationsmembran sowie ein Trägergas in Verbindung mit einem Sensor eingesetzt wird.

Der Erfindung liegt die Aufgabe zugrunde, die Verwendung einer Sonde zur Bestimmung insbesondere von flüchtigen Bestandteilen in einer wässrigen Lösung, bevorzugt der Alkoholkonzentration einer wässrigen Lösung, zu schaffen, die eine höhere Meßgenauigkeit und Meßgeschwindigkeit und einen langfristigen ungestörten Einsatz in technischen Prozessen mit kontinuierlicher Messung ohne Einsatz von Trägergas ermöglicht.

Diese Aufgabe wird erfindungsgemäß durch Verwendung, einer Sonde gemäß Anspruch 1 erreicht, die dadurch gekennzeichnet ist, daß der Abfluß von durch die Membran in die Meßkammer permeierenden Gase auf der der Membran abgewandten Seite des Sensors aus der Meßkammer direkt in die Atmosphäre über einen ständig offenen durchgehenden Druckentlastungskanal ausgebildet ist, und der Druckentlastungskanal an einem Ende über eine Öffnung des Gehäuses des Sensors an die Meßkammer angeschlossen ist und am anderen ausgangsseitigen Ende eine bezüglich der Größe definierte Auslaßöffnung aufweist, so daß der durch die Membran in die Meßkammer permeierende Volumenstrom der Gase stets etwas größer ist als der Volumenstrom der aus der Meßkammer über die Auslaßöffnung des Druckentlastungskanals in die Atmosphäre abfließenden Gase, wodurch der in der Meßkammer infolge der eindiffundierenden Gase sich aufbauende Partialdruck begrenzbar ist und die ständige zu der Alkoholkonzentration der zu messenden Flüssigkeit proportionale Aufkonzentrierung des Alkohols in der Meßkammer ermöglicht.

Erfindungsgemäß wird mittels des Druckentlastungskanals der Abfluß von Gasen aus der Meßkammer direkt in die Atmosphäre über den ständig offenen Druckentlastungskanal geschaffen. Dadurch wird es möglich, den sich in der Meßkammer infolge der durch die Membran eindiffundierenden Gase einstellenden und aufbauenden Partialdruck zu begrenzen. Infolge der durch die Membran aus der zu messenden Flüssigkeit in die Meßkammer permeierenden Gase, zum Beispiel Alkohol, Ethanol, erhöht sich die Gaskonzentration, zum Beispiel Alkoholkonzentration, in der Meßkammer proportional und ermöglicht ein genaues kontinuierliches Messen. Der erfindungsgemäß ermöglichte Abfluß der in die Meßkammer permeierenden Gase in einem geringeren Umfange als dem in die Meßkammer hineinpermeierenden Volumenstrom des Gases ermöglicht die ständige zu der Alkoholkonzentration der zu messenden Flüssigkeit proportionale Aufkonzentrierung des Alkohols in der Meßkammer. Überraschenderweise wurde festgestellt, daß durch die Ableitung der Gase aus der Meßkammer über einen separaten Druckentlastungskanal die Funktionstüchtigkeit der Sonde sowie die Meßleistung wesentlich verbessert werden. Es ist kein Trägergas mehr für die Spülung der Membran erforderlich. Die Sonde kann über lange Zeit störungsfrei arbeiten und in technischen Prozessen, wie Fermentationsprozessen, eingesetzt werden. Es ist auch nur eine kleine Membranfläche erforderlich. Ein weiterer Vorteil der Sonde besteht darin, daß diese nicht in die zu messende Flüssigkeit eingetaucht werden muß.

Vorteilhafte Ausgestaltungen der Erfindung sind den kennzeichnenden Merkmalen der Unteransprüche entnehmbar.

Erfindungsgemäß wird eine für die Pervaporation geeignete Membran für die Messung der Alkoholkonzentration in einer wässrigen Lösung benutzt.

Unter Pervaporation ist eine Kombination von Verdampfen und Membranpermeation zu verstehen. Hierbei handelt es sich um eine Membrantrenntechnik, bei der die eine Seite einer porenlosen Polymermembran - Pervaporationsmembran - mit einer flüssigen Mischung verschiedener Komponenten in Kontakt ist, während auf der anderen Membranseite das Permeat in der Dampfphase entfernt wird. Der transmembrane Fluß wird durch einen Partialdruckgradienten über die Membran verursacht.

Neben den bekannten Membranen auf Basis Silikon und/oder Polytetrafluorethylen, die sich für die Permeation von Alkoholen aus Alkohol/Wassergemischen bewährt haben, können erfindungsgemäß auch Mehrschichtmembranen eingesetzt werden, die eine höhere Trennleistung aufweisen.

Als geeignet für eine Trennwirkung, die sich auf Ethanol bezieht, haben sich Pervaporationsmembrane erwiesen, die auf Basis von aromatischen Polyimiden, Polyamiden, Polyamidimiden, Polysulfonen, Polyvinylchlorid, Polyacrylnitril, Polyacrylamide, Polyetheretherketon, Polyetherketon, Polyethersulfon oder Polyetherimid hergestellt sind und eine selektive Schicht auf Basis von Polysiloxanen, wie Polydimethylsiloxan oder Polyoctamethylcyclotetrasiloxan mit einer selektiven Schichtdicke von 10-20 µm aufweisen.

Die für die Erfindung einsetzbaren Halbleitergassensoren sind handelsüblich erhältlich, wie auch die eingangs der Beschreibung erwähnten europäischen Offenlegungsschriften beschreiben. Beispielsweise können für die Erfindung Halbleitergassensoren der Unitronic GmbH mit der Handelsbezeichnung TGS 822 eingesetzt werden.

Die erfindungsgemäße Verwendung zeichnet sich durch einen besonders einfachen Aufbau aus mit einem äußeren Sondenkörper mit einem durchgehenden Lumen und einem von einer Seite in das Lumen des Sondenkörpers einsetzbaren zweiten sogenannten inneren Körper. Bevorzugt sind sowohl der Sondenkörper als auch der innere Körper zylindrisch bzw. als rotationssymmetrische Teile ausgebildet, ebenso die Lumen und Bohrungen. Damit wird es möglich, in sehr einfacher Weise die Sonde durch Ineinanderstecken aufzubauen, wobei die Pervaporationsmembran nur noch als flache Scheibe ausgebildet ist und das Lumen an der Stirnseite des Sondenkörpers quer überspannend im Inneren des Sondenkörpers eine von der äußeren Atmosphäre und Umgebung abgetrennte Meßkammer bildet, in der der Sensor mit seinem Gehäuse angeordnet ist. Das Gehäuse des Sensors begrenzt die Meßkammer auf der der Membran gegenüberliegenden Seite. Durch die Pervaporationsmembran eindringende Gase gelangen durch die Meßkammer zu dem Sensor und lösen durch Widerstandsveränderung desselben ein Signal aus, das über Meßleitungen einer entsprechenden Auswerteelektronik, beispielsweise mit Mikroprozessor und Anzeigeeinheit zu geführt wird.

Der Abfluß der in die Meßkammer permeierenden Gase wird durch den Druckentlastungskanal ermöglicht, der bevorzugt durch den inneren Körper vom Gehäuse des Sensors bis nach außen geführt ist. Mit Hilfe des inneren Körpers kann zugleich die Position des Sensors bzw. dessen Gehäuse im Sondenkörper festgelegt werden. Der Druckentlastungskanal wird entlang seines Weges durch die Sonde an allen Übergängen, wie vom Gehäuse des Sensors zum Stecker des Sensors oder zum inneren Körper, mittels dazwischen angeordneter Dichtringe abgedichtet.

Der Druckentlastungskanal kann einen größeren Querschnitt als die Auslaßöffnung am Ende desselben aufweisen, um ein Luftpolster zu bilden.

Um einen einfachen Aufbau, Montage und Demontage, genaue Positionierung und auch leichte Reinigung der Sonde zu ermöglichen, wird vorgeschlagen, daß der Sondenkörper eine über die vordere Stirnseite des Sondenkörpers aufsetzbare und fixierbare Schraubkappe aufweist, die eine zentrale Durchtrittsöffnung aufweist. Es ist dann möglich, die Membran auf der vorderen Stirnseite des Sondenkörpers außen aufliegend anzuordnen und mittels der Schraubkappe gegebenenfalls unter Zwischenlage eines Dichtringes zu fixieren.

So ist die Pervaporationsmembran zwischen Stirnseite und Schraubkappe fixiert, während der Sensor innerhalb des Sondenkörpers im Lumen in einer bestimmten Position eingesetzt und gehaltert ist. Die Abfuhr der Gase aus der Meßkammer erfolgt durch den inneren Körper hindurch, der mit einem durchgängigen Kanal und einer definierten Auslaßöffnung versehen ist. Die Auslaßöffnung des Druckentlastungskanals ist stets kleiner als die von der Membran abgedeckte Eintrittsbohrung der Sonde zur Meßkammer. Bevorzugt kann die Auslaßöffnung weniger als 10 % der Größe dieser Eintrittsbohrung betragen. Der in die Meßkammer permeierende Volumenstrom an Gas wird durch die Größe der wirksamen Membranfläche, die mit der zu messenden Flüssigkeit in Kontakt kommt und von der Trennleistung der Membran, also den Membran-Werkstoffen bestimmt. Die Größe, d.h. der Austrittsquerschnitt der Auslaßöffnung, ist so zu wählen, daß der durch die Auslaßöffnung in die Atmosphäre abströmende Volumenstrom stets kleiner als der in die Meßkammer permeierende Gasvolumenstrom gehalten wird, so daß die Aufkonzentrierung des Alkohols bzw. des zu messenden Gases in der Meßkammer sichergestellt ist. Auf diese Weise sind stets definierte Volumenverhältnisse in der Meßkammer herstellbar, die ein genaues Messen der Alkoholkonzentration in einer wässrigen Flüssigkeit mittels durch die Pervaporationsmembran permeierenden Alkohols mit dem Sensor ermöglichen. Die Auslaßöffnung muß so klein sein, daß sie einen durch das Eindiffundieren der Gase durch die Membran in der Meßkammer sich einstellenden ausreichenden Partialdruckaufbau ermöglicht.

Der Aufbau des Körpers der Sonde mit im wesentlichen rotationssymmetrischen Teilen ermöglicht eine wirtschaftliche Herstellung derselben und eine einfache Montage und Demontage auch zur Reinigung der Sonde. Außerdem sind die Position der Membran und des Sensors zueinander genau festlegbar und damit die Größe der Meßkammer definiert und festlegbar, ebenso die für den Gasaustausch durch Diffusion zur Verfügung stehende mittels der Eintrittsbohrung in das Lumen des Sondenkörpers freigegebene wirksame Membranfläche.

Die Erfindung und ihre weitere Ausgestaltung wird nachfolgend in der Zeichnung anhand von Ausführungsbeispielen erläutert. Es zeigen
- Fig. 1: eine Sonde zum Messen der Alkoholkonzentration mit einem einteiligen äußeren Sondenkörper in schematischer Darstellung im Längsschnitt und
- Fig. 2: eine Sonde zum Messen der Alkoholkonzentration mit mehrteiligem Aufbau mit Sondenkörper im schematischen Längsschnitt.

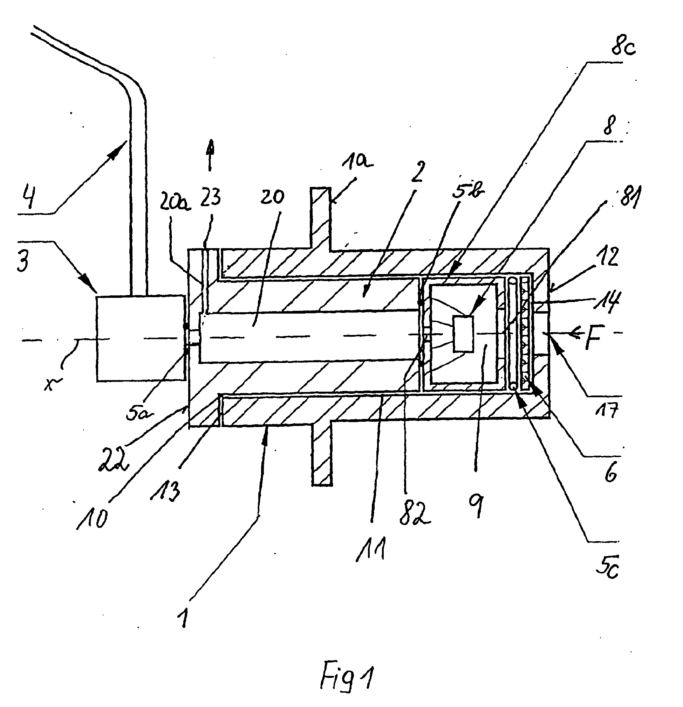

Die Sonde nach Fig. 1 hat einen zylindrischen rohrförmig ausgebildeten Sondenkörper 1, der an seiner vorderen Stirnseite 12 mittels einer Wandung teilweise verschlossen ist und in der Wandung eine zentrale Eintrittsbohrung 17 aufweist. Der innere Hohlraum des Sondenkörpers 1 bildet das Lumen 11, das von der Eintrittsbohrung 17 bis zur rückwärtigen Stirnseite 13 des Sondenkörpers durchgehend ausgebildet ist. Im Bereich der vorderen Stirnseite 12 des Sondenkörpers ist die als flache Scheibe ausgebildete Pervaporationsmembran 6 quer zum Lumen 11 verlaufend und quer zur Längsachse X des Sondenkörpers 1 verlaufend innerhalb des Sondenkörpers 1 angeordnet, so daß das Lumen 11 nach außen durch die Membran 6 abgetrennt ist. Die Membran liegt hier an der Innenseite der vorderen stirnseitigen Wandung 12 des Sondenkörpers 1 an. Die Eintrittsbohrung 17 wird von der Membran 6 abgedeckt und die Größe der Eintrittsbohrung 17 bestimmt die aktive für die Diffusion zur Verfügung stehende wirksame Membranfläche. Die Sonde wird mit ihrem vorderen Ende mit der zu messenden Flüssigkeit F in Kontakt gebracht, die durch die Eintrittsbohrung 17 hindurch auf die Membran 6 trifft.

Unter Zwischenlage eines Dichtringes 5c ist das Gehäuse 8c mit dem im Inneren angeordneten Halbleitergassensor 8 in dem Lumen 11 angeordnet. Das Gehäuse 8c des Sensors weist auf der der Membran 6 zugewandten Seite eine Eingangsöffnung 81 und auf der gegenüberliegenden Seite eine Austrittsöffnung 82 auf, die bevorzugt koaxial zur Längsachse X des Sondenkörpers angeordnet sind. Der Halbleitergassensor 8 ist innerhalb des Gehäuses angeordnet und aufgehängt. Die elektrischen Verbindungen werden zum rückwärtigen Ende der Sonde einem am hinteren Ende des inneren Körpers 2 angebrachten Stecker 3 zugeführt, dessen Leitungen 4 zu einer Auswerteeinheit und Anzeigeeinheit führen. Als Halbleitergassensor kann beispielsweise der bereits eingangs genannte Sensor TGS 822 von Unitronic GmbH eingesetzt werden.

Die mit Luft gefüllte Meßkammer 9 ist innerhalb des Gehäuses 8c des Sensors bis zur Membran 6 hin gebildet. Auf der der Membran 6 abgewandten Seite des Gehäuses 8c des Sensors schließt sich der in das Lumen 11 von der rückwärtigen Stirnseite 13 des Sondenkörpers 1 eingesetzte innere Körper 2 an, der ebenfalls als zylindrischer Körper ausgebildet ist und eine durchgehende Bohrung aufweist, die den Druckentlastungskanal 20 bildet und an die Austrittsöffnung 82 des Gehäuses 8c anschließt. Im Bereich des rückwärtigen Endes der Sonde bzw. des inneren Körpers 2 weist der Druckentlastungskanal eine kleine Auslaßöffnung 23 definierter Größe auf, die beispielsweise als seitliche Bohrung 20a durch den inneren Körper 2 nach außen geführt ist. Die Auslaßöffnung 23 kann auch durch den Stecker 3 geführt sein. Die Auslaßöffnung 23 ist sehr klein auszubilden, insbesondere ist sie wesentlich kleiner als die Eintrittsbohrung 17 auszubilden, da sie lediglich der Ableitung der in die Meßkammer permeierenden Gase in die Atmosphäre dient.

Bei der Sonde ist die als flache Scheibe ausgebildete Pervaporationsmembran 6 mit dem Halbleitergassensor 8 so kombiniert, daß durch die Anordnung der als flache Scheibe ausgebildeten Membran 6 am Ende des durch das Lumen 11 offenen zylindrischen Sondenkörpers 1 ein mit Luft gefüllter Raum im Inneren des Sondenkörpers von der zu analysierenden Flüssigkeit F abgetrennt ist. Dieser mit Luft gefüllte Raum wird durch das Gehäuse 8c des Sensors auf der der Membran abgewandten Seite begrenzt und bildet die Meßkammer 9. Die Größe der Meßkammer 9 wird durch das Innere des Gehäuses 8c des Sensors und den zwischen Membran 6 und Vorderseite des Gehäuses mit Eintrittsöffnung 81 noch verbleibenden Raum 9a des Lumens 11 bestimmt.

Der Halbleitergassensor 8 spricht durch Veränderung seines Widerstandes, insbesondere Verringerung desselben mit dem Ansteigen von Gas- oder Dampfkonzentration in der Meßkammer an. Die Widerstandsveränderung löst ein elektrisches Signal aus, so daß die sich verändernde zu messende Alkoholkonzentration in der Meßkammer 9 durch den durch die Membran 6 aus der Flüssigkeit F permeierenden Alkohol gemessen werden kann.

Das Gehäuse 8c mit dem Sensor 8 ist so in dem Sondenkörper 1, d.h. in dessen Lumen 11, untergebracht und wird von dem zweiten in den Sondenkörper 1 eingeführten inneren Körper 2 positioniert, daß das Gehäuse mit der Vorderseite unter Zwischenlage des Dichtringes 5c die Membran 6 gegen das stirnseitige vordere Ende innenseitig am Sondenkörper drückt und festlegt. Das Gas kann aus der Meßkammer 9 nur durch die am hinteren Ende des inneren Körpers 2 befindliche Auslaßbohrung 23 über die Austrittsöffnung 82 des Gehäuses und den Druckentlastungskanal 20 des inneren Körpers in die Atmosphäre abströmen. Innerhalb der Meßkammer 9 entsteht so ein Gasgemisch, dessen Alkohlgehalt in ständigem Diffusionsgleichgewicht mit der zu messenden Flüssigkeit F steht. Die Alkoholkonzentration der Meßkammeratmosphäre erzeugt die Widerstandsveränderung an der Oberfläche des Gassensors 8. Diese Widerstandsveränderung kann mit Hilfe geeigneter Algorithmen in eine mathematische Beziehung mit der Alkoholkonzentration gebracht werden, d.h. das Meßsignal wird auf einen Rechner gegeben.

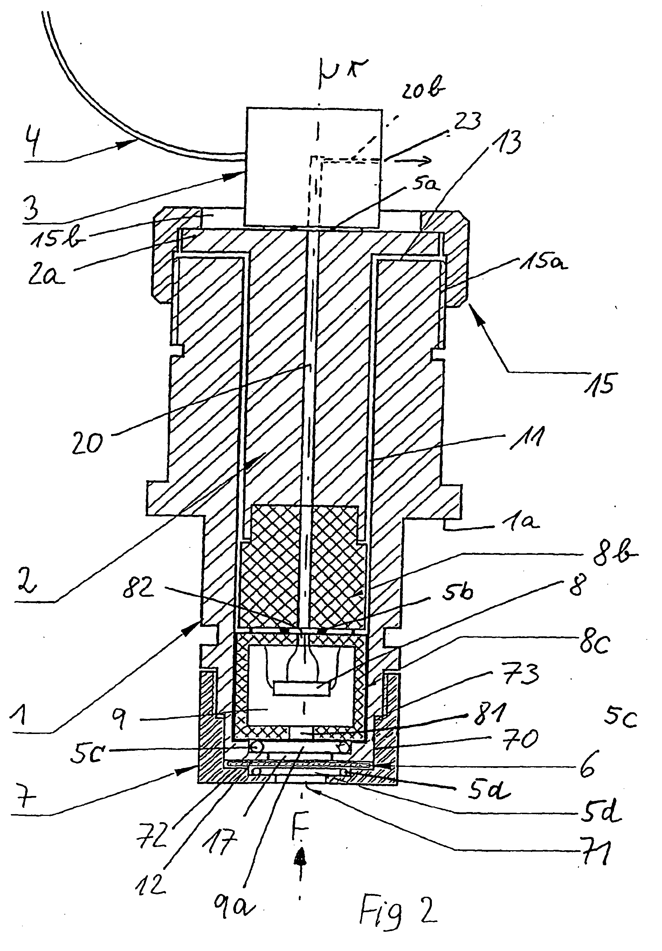

In der Fig. 2 ist eine Abwandlung der Sonde nach Fig. 1 dargestellt, bei der die Sonde mehrteilig ausgebildet ist, wobei an dem vorderen stirnseitigen Ende 13 des Sondenkörpers 1 eine Schraubkappe 7 aufgebracht ist. Die Schraubkappe 7 weist ebenfalls an ihrer Kopfseite eine Eintrittsöffnung 71 auf.

Mit Hilfe der Schraubkappe 7 ist es möglich, die Membran 6 auf der vorderen Außenseite der Stirnseite 12 des Sondenkörpers als flache Scheibe aufzulegen und sie mittels der darüber gesetzten Schraubkappe 7 zu fixieren. Bevorzugt wird noch ein Dichtring 5d zwischen der Schraubkappe und der Membran eingelegt. Diese Bauweise der Sonde ermöglicht ein einfaches Reinigen oder Auswechseln der Membran 6. Die zu messende Flüssigkeit F kann auch hier durch die Öffnung 71 in Pfeilrichtung P1 direkt mit der Membran 6 kontaktieren, während die Gase auf der anderen Seite durch die Eintrittsbohrung 17 des Sondenkörpers in die Meßkammer gelangen.

Der Sensor 8 mit seinem Gehäuse 8c ist im Inneren des Sondenkörpers 1 in dem Lumen untergebracht, und zwar direkt anschließend an die Innenseite der Stirnwandung 12 des Sondenkörpers unter Zwischenlage eines Dichtringes 5c. Der Sensor kann zusätzlich mit einem Sensorstecker 8b auf seiner rückwärtigen Gehäuseseite ausgerüstet sein, wobei der Stecker 8b ebenfalls unter Zwischenlage eines Dichtringes 5b mit dem Gehäuse 8c verbunden ist. An den Stecker 8b schließt sich der innere Körper 2 an, der vom rückwärtigen Ende 13 des Sondenkörpers in das Lumen eingebracht ist und der die Position des Sensors mit Gehäuse 8c und Stecker 8b festlegt. Der innere Körper 2 ist beispielsweise an seinem rückwärtigen Ende mit einem seitlichen am Sondenkörper stirnseitig aufliegenden Flansch 2a ausgebildet und wird mittels der Überwurfkappe 15 mit Schraubgewinde 15a an dem Sondenkörper 1 befestigt. Auch die Überwurfkappe 15 hat kopfseitig eine Durchtrittsöffnung 15b. Am rückwärtigen Ende des inneren Körpers 2 ist der Anschlußstecker 3 mit Leitungen 4 für die elektrische Verbindung mit dem Sensor 8 angebracht, der durch die Öffnung 15b der Überwurfkappe gesteckt ist. Der Druckentlastungskanal 20 ist im Anschluß an die Austrittsöffnung 82 des Gehäuses 8c des Sensors durch den Stecker 8b des Sensors und den inneren Körper 2 sowie durch den Stecker 3 bis zur Auslaßöffnung 23 durchgängig geführt. Der Druckentlastungskanal 20 ist stets offen, d.h. die Auslaßöffnung 23 wird nicht verschlossen. So können die zu messenden Gase aus der Flüssigkeit F durch Kontaktierung mit der Membran 6 in die Meßkammer 9 zu dem Sensor 8 gelangen, hier erfaßt werden und die erzeugten Signale dann zur Auswerteeinheit weitergeleitet werden. Aus der Meßkammer 9 kann dann über den nach hinten zum Sondenende hinausgeführten Druckentlastungskanal 20 der gewünschte Abfluß der in die Meßkammer permeierenden Gase in die Atmosphäre erfolgen.

Der Sondenkörper 1 weist außenseitig einen Flanschvorsprung 1a auf, mit dem er an einer Wandung oder dergleichen zur Anlage gebracht werden kann.

Der Druckentlastungskanal 20 ist durchgängig bis zum hinteren Ende jeweils im Übergang der aneinandergereihten Bauteile mittels dazwischengelegter Dichtringe 5b zwischen Sensorgehäuse 8c und Sensorstecker 8b bzw. innerem Körper 2 und Stecker 3 mittels Dichtring 5a abgedichtet.

Die Sonde gemäß Fig. 2 zeichnet sich durch einen einfachen Aufbau im wesentlichen rotationssymmetrisch ausgebildeter Teile aus, die einfach miteinander zu verbinden sind. Die Positionierung und Festlegung der Membran erfolgt mittels der Schraubkappe am vorderen Ende des Sondenkörpers, während die Positionierung und Festlegung des Sensors mit Gehäuse mittels des inneren in das Lumen des Sondenkörpers eingesetzten Körpers 2 erfolgt. Der Druckentlastungskanal 20 ist bevorzugt zentrisch koaxial zur Längsachse X der Sonde geführt, und zwar bis in den Stecker 3 hinein und dort mit einer Auslaßöffnung 23 über eine seitliche Bohrung 20b versehen.

Die Verwendung der Sonde ermöglicht eine hohe Meßgenauigkeit bei einer ausreichend schnellen Arbeitsweise und arbeitet störungsfrei über eine lange Zeit hinweg, so daß sie mit Vorteil in technischen Prozessen zur Überwachung von Produktionsprozessen einsetzbar ist.

Die Sonde ist bezüglich der Größe der Meßkammer, der Größe der wirksamen Membranfläche und des Umfanges des Abflusses der Gase aus der Meßkammer in die Atmosphäre über die Auslaßöffnung des Druckentlastungskanals reproduzierbar herstellbar, auch bei Montage und nach Demontage, wodurch stets ein einwandfreies Messen ermöglicht ist.

## Patentansprüche

1. Verwendung einer Sonde zum Messen von flüchtigen Bestandteilen in einer wässrigen Lösung, wie der Bestimmung der Alkoholkonzentration einer wässrigen Lösung, mit einem Sondenkörper (1) mit einem durchgehenden Lumen (11) und einer quer zum Lumen verlaufend angeordneten Membran (6), die das Lumen nach außen hin abtrennt und mit einem innerhalb des Lumens mit Abstand zu der Membran in einem Gehäuse (8c) angeordneten Halbleitergassensor (8), wobei eine von der äußeren Atmosphäre und Umgebung abgetrennte mit Luft gefüllte Meßkammer (9) innerhalb des Gehäuses (8c) bis zur Membran (6) hin ausgebildet ist, die über einen Druckentlastungskanal (20) mit der äußeren Atmosphäre verbunden ist und der Sensor (8) auf die durch Membran (6) in die Meßkammer (9) permeierenden Gase mit einer Änderung des elektrischen Widerstandes anspricht, **dadurch gekennzeichnet, dass** der Druckentlastungskanal (20) ständig offen ausgebildet ist und ein Abfluss von durch die Membran (6) in die Meßkammer (9) permeierenden Gase auf der Membran abgewandten Seite des Sensors (8) aus der Meßkammer (9) direkt in die Atmosphäre über den ständig offenen durchgehenden Druckentlastungskanal (20) bewirkt wird, und der Druckentlastungskanal (20) an einem Ende über eine Öffnung (82) des Gehäuses (8c) des Sensors (8) an die Meßkammer (9) angeschlossen ist und am anderen ausgangsseitigen Ende eine bezüglich der Größe definierte Auslaßöffnung (23) aufweist, so dass der durch die Membran (6) in die Meßkammer (9) permeierende Volumenstrom der Gase stets etwas größer ist als der Volumenstrom der aus der Meßkammer (9) über die Auslaßöffnung (23) des Druckentlastungskanals (20) in die Atmosphäre abfließenden Gase, wodurch der in der Meßkammer (9) infolge der eindiffundierenden Gase sich aufbauende Partialdruck begrenzbar ist und die ständige zu der Alkoholkonzentration der zu messenden Flüssigkeit proportionale Aufkonzentrierung des Alkohols in der Meßkammer ermöglicht.

2. Verwendung nach Anspruch 1,
**dadurch gekennzeichnet, daß** auf der der Membran (6) abgewandten Seite des Sensors (8) bzw. des Gehäuses (8c) des Sensors ein innerer Körper (2) vorgesehen und in das Lumen (11) des Sondenkörpers (1) von der rückwärtigen Stirnseite des Sondenkörpers (1) eingeführt ist, wobei der innere Körper (2) in Wirkverbindung mit dem Gehäuse (8c) des Sensors steht, dergestalt, daß er zumindest das Gehäuse (8c) mit dem Sensor (8) positioniert und der Druckentlastungsskanal (20) durch den inneren Körper (2) geführt ist.

3. Verwendung nach Anspruch 1 oder 2,
**dadurch gekennzeichnet, daß** der Sondenkörper (1) an seinem vorderen stirnseitigen Ende (12) durch Ausbildung einer Stirnwand eine gegenüber dem Lumen (11) verkleinerte Eintrittsbohrung (17) aufweist und die Membran (6) auf der Außenseite der Stirnwand oder auf der Innenseite der Stirnwand gegebenenfalls unter Zwischenlage eines Dichtringes (5c) die Eintrittsbohrung (17) abdeckend angeordnet ist und der Querschnitt der Auslaßöffnung (23) des Druckentlastungskanals (20) kleiner als der Querschnitt der Eintrittsbohrung (17) ausgebildet ist.

4. Verwendung nach einem der Ansprüche 1 bis 3,
**dadurch gekennzeichnet, daß** der Sondenkörper (1) mit Lumen (11) als Hohlzylinder und der innere Körper (2) als Zylinderkörper mit axial durchgehendem Druckentlastungskanal (20) ausgebildet sind.

5. Verwendung nach einem der Ansprüche 1 bis 2,
**dadurch gekennzeichnet, daß** der Sondenkörper (1) eine über die vordere Stirnseite des Sondenkörpers (1) aufsetzbare und fixierbare Schraubkappe (7) aufweist, die eine zentrale Durchtrittsöffnung (71) aufweist und die Membran (6) auf der vorderen Stirnseite (12) des Sondenkörpers (1) außen aufliegend angeordnet und mittels der Schraubkappe (7) gegebenenfalls unter Zwischenlage eines Dichtringes (5d) fixiert ist.

6. Verwendung nach einem der Ansprüche 1 bis 5,
**dadurch gekennzeichnet, daß** am rückwärtigen Ende des Sondenkörpers (1) eine Überwurfkappe (15) mit Schraubgewinde (15a) und einer kopfseitigen Durchtrittsöffnung (15b) zur Befestigung des inneren Körpers (2) am Sondenkörper (1) vorgesehen ist.

7. Verwendung nach einem der Ansprüche 1 bis 6,
**dadurch gekennzeichnet, daß** am rückwärtigen Ende des inneren Körpers (2) ein Stecker (3) für die elektrische Verbindung mit dem Sensor (8) angeordnet ist und der Druckentlastungskanal (20) im Anschluß von dem inneren Körper (2) durch den Stecker (3) bis zur Auslaßöffnung (23) weitergeführt ist.

8. Verwendung nach einem der Ansprüche 1 bis 7,
**dadurch gekennzeichnet, daß** zwischen dem Gehäuse (8c) des Sensors und dem inneren Körper (2) bzw. einem an das Gehäuse (8c) des Sensors anschließenden Sensorstecker (8b) ein den Druckentlastungskanal (20) umgebender Dichtungsring (5b) angeordnet ist.

9. Verwendung nach einem der Ansprüche 1 bis 8,
**dadurch gekennzeichnet, daß** zwischen dem rückwärtigen stirnseitigen Ende des inneren Körpers (2) und dem aufgesetzten Stecker (3) ein den Druckentlastungskanal (20) umgebender Dichtungsring (5a) vorgesehen ist.

10. Verwendung nach einem der Ansprüche 1 bis 9,
**dadurch gekennzeichnet, daß** das Gehäuse (8c) des Sensors auf der der Membran (6) zugewandten Seite eine Eingangsöffnung (81) aufweist, die symmetrisch zur Längsachse (X) des zylindrischen Sondenkörpers ebenso wie die Eintrittsbohrung (17) des Sondenkörpers (1) und der Druckentlastungskanal (20) ausgebildet ist.

11. Verwendung nach einem der Ansprüche 1 bis 10,
**dadurch gekennzeichnet, daß** eine mehrschichtige Pervaporations-membran eingesetzt ist, deren stoffspezifische Trennwirkung In Bezug auf Ethanol ausgelegt ist.

12. Verwendung nach einem der Ansprüche 1 bis 10,
**dadurch gekennzeichnet, daß** der Querschnitt des Druckentlastungskanals (20) größer als der Querschnitt der Auslaßöffnung (23) ist.

## Claims

1. Use of a probe for measuring volatile components in an aqueous solution, as when determining the alcohol concentration of an aqueous solution, having a probe body (1) with a continuous lumen (11) and a membrane (6) which is arranged in a fashion running transverse to the lumen and separates the lumen from the outside, and having a semiconductor gas sensor (8) arranged inside the lumen at a spacing from the membrane in a housing (8c), a measuring chamber (9) which is separated from the outer atmosphere and surroundings and is filled with air being constructed inside the housing (8c) up to the membrane (6), the measuring chamber (9) being connected via a pressure-relief duct (20) with the outer atmosphere, and the sensor (8) responding to the gases permeating through the membrane (6) into the measuring chamber (9) with a change in the electric resistance, **characterized in that** the pressure-relief duct (20) is formed permanently open and an outflow of gases, permeating through the membrane (6) into the measuring chamber (9) on the side of the sensor (8) averted from the membrane from the measuring chamber (9) directly into the atmosphere is effected via the permanently open continuous pressure-relief duct (20) and the pressure-relief duct (20) is connected at one end to the measuring chamber (9) via an opening (82) in the housing (8c) of the sensor (8) and has at the other end, on the outgoing side, an outlet opening (23) defined with reference to size such that the volumetric flow of the gases which permeates through the membrane (6) into the measuring chamber (9) is always somewhat greater than the volumetric flow of the gases flowing off into the atmosphere from the measuring chamber (9) via the outlet opening (23) in the pressure-relief duct (20), as a result of which the partial pressure building up in the measuring chamber (9) as a consequence of the gases diffusing in can be limited and enables the alcohol in the measuring chamber to be continuously concentrated in a fashion proportional to the alcohol concentration of the liquid to be measured.

2. Use of a probe according to claim 1, **characterized in that** an inner body (2) is provided on the side of the sensor (8), or of the housing (8c) of the sensor, averted from the membrane (6) and is inserted into the lumen (11) of the probe body (1) from the rear end face of the probe body (1), the inner body (2) being operatively connected to the housing (8c) of the sensor in such a way that it at least positions the housing (8c) with the sensor (8), and the pressure-relief duct (20) is led through the inner body (2).

3. Use of a probe according to claim 1 or 2, **characterized in that**, through the formation of an end wall, the probe body (1) has at its front face end (12) an inlet bore (17) which is reduced by comparison with the lumen (11), and the membrane (6) is arranged in a fashion covering the inlet bore (17) on the outside of the end wall or on the inside of the end wall, if appropriate with the interposition of a sealing ring (5c), and the cross section of the outlet opening (23) of the pressure-relief duct (20) is designed to be smaller than the cross section of the inlet bore (17).

4. Use of a probe according to one of claims 1 to 3, **characterized in that** the probe body (1) with lumen (11) is constructed as a hollow cylinder, and the inner body (2) is constructed as a cylindrical body with an axially continuous pressure-relief duct (20).

5. Use of a probe according to one of claims 1 to 2, **characterized in that** the probe body (1) has a screw cap (7) which can be mounted over the front end face of the probe body (1) and fixed and which has a central passage opening (71), and the membrane (6) is arranged resting outside on the front end face (12) of the probe body (1) and is fixed by means of the screw cap (7), if appropriate with the interposition of a sealing ring (5d).

6. Use of a probe according to one of claims 1 to 5, **characterized in that** a union cap (15) with a screw thread (15a) and a head-side passage opening (15b) is provided at the rear end of the probe body (1) in order to fasten the inner body (2) on the probe body (1).

7. Use of a probe according to one of claims 1 to 6, **characterized in that** a plug (3) is arranged at the rear end of the inner body (2) for the electric connection to the sensor (8) and, following on from the inner body (2), the pressure-relief duct (20) is led further through the plug (3) up to the outlet opening (23).

8. Use of a probe according to one of claims 1 to 7, **characterized in that** a sealing ring (5b) surrounding the pressure-relief duct (20) is arranged between the housing (8c) of the sensor and the inner body (2) or a sensor plug (8b) connected to the housing (8c) of the sensor.

9. Use of a probe according to one of claims 1 to 8, **characterized in that** a sealing ring (5a) surrounding the pressure-relief duct (20) is provided between the rear face end of the inner body (2) and the mounted plug (3).

10. Use of a probe according to one of claims 1 to 9, **characterized in that**, on the side facing the membrane (6), the housing (8c) of the sensor has an inlet opening (81) which is designed symmetrically relative to the longitudinal axis (X) of the cylindrical probe body, just as are the inlet bore (17) of the probe body (1) and the pressure-relief duct (20).

11. Use of a probe according to one of claims 1 to 10, **characterized in that** use is made of a multilayer pervaporation membrane whose substance-specific separating action is designed with reference to ethanol.

12. Use of a probe according to one of claims 1 to 10, **characterized in that** the cross section of the pressure-relief duct (20) is greater than the cross section of the outlet opening (23).

## Revendications

1. Utilisation d'une sonde pour la mesure de composants volatiles dans une solution aqueuse, comme pour effectuer la détermination de la concentration en alcool d'une solution aqueuse, comprenant un corps de sonde (1) avec un alésage continu (11) et une membrane disposée perpendiculairement à celui-ci qu'elle sépare de l'extérieur, un capteur à semi-conducteur (8) logé dans un boîtier (8c) qui est monté dans l'alésage à une certaine distance de la membrane et à l'intérieur duquel se trouve une chambre de mesure (9), étant séparée de l'atmosphère extérieure et de l'environnement, remplie d'air et allant jusqu'à la membrane (6), la chambre de mesure (9) étant reliée avec l'atmosphère extérieure par un canal de décharge de pression (20), le capteur (8) répondant aux gaz qui pénètrent dans la chambre de mesure à travers la membrane (6), par une variation de sa résistance électrique, **caractérisée en ce que** le canal de décharge de pression (20) est constitué constamment ouvert et un déversoir des gaz qui pénètrent dans la chambre de mesure (9) à travers la membrane (6) et en sortent directement à l'atmosphère du côté du capteur (8) éloigné de la membrane, est obtenu par le canal de décharge de pression (20) constamment ouvert et qui est raccordé à une extrémité, à la chambre de mesure (9) par une ouverture (82) du boîtier (8c) du capteur (8), tandis qu'à son autre extrémité, située du côté sortie, ce canal présente une ouverture d'évacuation (23) définie, en ce qui concerne sa grandeur, de sorte que le courant volumique des gaz qui pénètrent dans la chambre de mesure (9) à travers la membrane (6) est toujours un peu plus grand que le courant volumique des gaz qui, sortant de la chambre de mesure (9), s'écoulent à l'atmosphère à travers l'ouverture d'évacuation (23) du canal de décharge de pression (20), de sorte que la pression partielle qui s'établit dans la chambre de mesure (9) du fait des gaz qui y pénètrent, peut être limitée, ce qui permet de concentrer l'alcool dans la chambre de mesure de manière constamment proportionnelle à la concentration que présente l'alcool dans le liquide objet de la mesure.

2. Utilisation d'une sonde selon la revendication 1, **caractérisée en ce que** du côté du capteur (8), c'est-à-dire du côté de son boîtier (8c) éloigné de la membrane (6), est prévu un corps interne (2) qui est introduit dans l'alésage (11) du corps de sonde (1) par sa face frontale arrière, ce corps interne (2) coopérant avec le boîtier (8c) du capteur, de manière à positionner au moins le boîtier (8c) avec le capteur (8), et à faire passer le canal de décharge de pression (20) à travers ce corps interne (2).

3. Utilisation d'une sonde selon la revendication 1 ou 2, **caractérisée en ce que** le corps de sonde (1) à son extrémité frontale avant (12), présente une paroi latérale percée d'une ouverture d'entrée (17) plus petite que l'alésage (11), et la membrane (6), sur la face externe ou sur la face interne de cette paroi, éventuellement avec interposition d'une bague d'étanchéité (5c), recouvre l'ouverture d'entrée (17), la section de l'ouverture d'évacuation (23) du canal de décharge de pression (20) étant plus petite que la section de l'alésage d'entrée (17).

4. Utilisation d'une sonde selon une des revendications 1 à 3, **caractérisée en ce que** le corps de sonde (1) présente un alésage (11) qui a la forme d'un cylindre creux et le corps interne (2) est un corps cylindrique traversé axialement par le canal de décharge de pression (20).

5. Utilisation d'une sonde selon une des revendications 1 à 2, **caractérisée en ce que** le corps de sonde (1) présente un capot vissable (7) qui peut être monté et fixé sur la face frontale avant du corps de sonde (1) et qui présente une ouverture de passage centrale (71), la membrane (6) étant montée à l'extérieur sur la face frontale avant (12) du corps de sonde (1) en étant fixée par le capot vissable (7) avec éventuellement interposition d'une bague d'étanchéité (5d).

6. Utilisation d'une sonde selon une des revendications 1 à 5, **caractérisée en ce qu'**à l'extrémité arrière du corps de sonde (1) est prévu un capot en forme d'écrou (15) présentant un filetage (15a) et du côté de la tête une ouverture de passage (15b) pour fixer le corps interne (2) sur le corps de sonde (1).

7. Utilisation d'une sonde selon une des revendications 1 à 6, **caractérisée en ce qu'**à l'extrémité arrière du corps interne (2) se trouve un connecteur (3) pour le raccordement électrique au capteur (8) et le canal de décharge de pression (20), à la suite du corps interne (2), se prolonge à travers le connecteur (3) jusqu'à l'ouverture d'évacuation (23).

8. Utilisation d'une sonde selon une des revendications 1 à 7, **caractérisée en ce qu'**entre le boîtier (8c) du capteur et le corps interne (2) ou un connecteur de capteur (8b) le raccordant au boîtier (8c) du capteur, est montée une bague d'étanchéité (5b) entourant le canal de décharge de pression (20).

9. Utilisation d'une sonde selon une des revendications 1 à 8, **caractérisée en ce qu'**entre l'extrémité frontale arrière du corps interne (2) et le connecteur monté (3), est prévue une bague d'étanchéité (5a) entourant le canal de décharge de pression (20).

10. Utilisation d'une sonde selon une des revendications 1 à 9, **caractérisée en ce que** le boîtier (8c) du capteur présente sur son côté en regard de la membrane (6) une ouverture d'entrée (81) qui, comme l'ouverture d'entrée (17) du corps de sonde (1) et le canal de décharge de pression (20), est de révolution autour de l'axe longitudinal (X) du corps de sonde cylindrique (1).

11. Utilisation d'une sonde selon une des revendications 1 à 10, **caractérisée en ce qu'**il est fait usage d'une membrane de pervaporation à plusieurs couches, dont le pouvoir de séparation spécifique à la matière est prévu par rapport à l'éthanol.

12. Utilisation d'une sonde selon une des revendications 1 à 10, **caractérisée en ce que** la section du canal de décharge de pression (20) est supérieure à la section de l'ouverture d'évacuation (23).
